Europäisches Patentamt

European Patent Office

Office européen des brevets

(11) Publication number: **0 389 453**
**A1**

## (12) EUROPEAN PATENT APPLICATION

(21) Application number: **90830121.1**

(22) Date of filing: **22.03.90**

(51) Int. Cl.5: **A61B 1/24, A61B 1/04**

(30) Priority: **22.03.89 IT 937789**

(43) Date of publication of application:
**26.09.90 Bulletin 90/39**

(84) Designated Contracting States:
**AT BE CH DE DK ES FR GB GR LI LU NL SE**

(71) Applicant: **Cozzi, Gualtiero**
**Via Francesco Nullo 13**
**I-50137 Firenze(IT)**

(72) Inventor: **Cozzi, Gualtiero**
**Via Francesco Nullo 13**
**I-50137 Firenze(IT)**

(74) Representative: **Mannucci, Gianfranco,**
**Dott.-Ing.**
**Ufficio Tecnico Ing. A. Mannucci Via della**
**Scala 4**
**I-50123 Firenze(IT)**

(54) **Fibre optical dental endoscope.**

(57) An endoscope consists of a handle (25) support-
ing an end part (27) which is introduced into the oral
cavity by means of a rigid tubular element (31). The
tubular element (31) contains two coaxial bundles of
optical fibres (37, 39) serving to illuminate the area
to be inspected and to conduct image information to
camera means (17, 21), respectively.

Fig. 1

EP 0 389 453 A1

# FIBER OPTICAL DENTAL ENDOSCOPE

In order to follow operations inside the mouth of the patient, the doctor at present uses a small angled mirror supported on the end of a shaft, with which it is possible to obtain an adequate view even of areas of the mouth which are otherwise not directly visible. Use of the mirror is not always practical and is frequently unsatisfactory.

The aim of the invention is to produce a device which renders more easy and convenient, and above all more complete, the possibility of checking the whole of the internal space of the mouth of the patient, even in areas to which it is very difficult to gain access and which are very difficult to inspect, such as, for example, root canals and deep caries.

According to the invention, these and other aims, which will prove evident to experts in the field from reading the following text, are achieved with an endoscope for dental use, consisting of a handle with an end part, to be introduced into the oral cavity, at the end of which look out fiber-optical means for illumination of the area to be inspected and for collecting the image to be conveyed to means for picking up the image.

The endoscope thus produced can replace the conventional small mirror for observation of the work and, in addition to making it possible to work in a more convenient position than that which comes about with the use of the small mirror, also constitutes an efficient analysis instrument. By virtue of its small diameter, the end part for observation in fact makes it possible to observe even the inside of a tooth for the examination of caries and root canals before and during the execution of the work.

In a practical embodiment, the end part of the handle is curved in order to facilitate access to the oral cavity and can, if necessary, also be made flexible so as to assume different angular positions in relation to the axis of said handle. In the latter case, means for determining the angular position assumed by said end part in relation to said handle can be provided on the handle.

Advantageously, the fiber-optical means can consist of a light guide with two coaxial bundles of optical fibers, a first bundle conveying light from a light source to the area to be inspected and a second (in general internal) bundle conveying the image from the area to be inspected to the means for picking up the image.

The invention will be better understood by following the description and the attached drawing, which shows a non-limitative practical exemplary embodiment of the invention itself. In the drawing,

Fig. 1 shows a basic diagram of the endoscope according to the invention;

Fig. 2 shows a wiring diagram of the system incorporating the endoscope;

Fig. 3 shows a lateral view of the endoscope proper;

Fig. 4 shows a detail of that end of the endoscope which is intended to be introduced into the mouth of the patient, in an improved embodiment, and

Fig. 5 shows an enlarged local cross-section of the end of the endoscope.

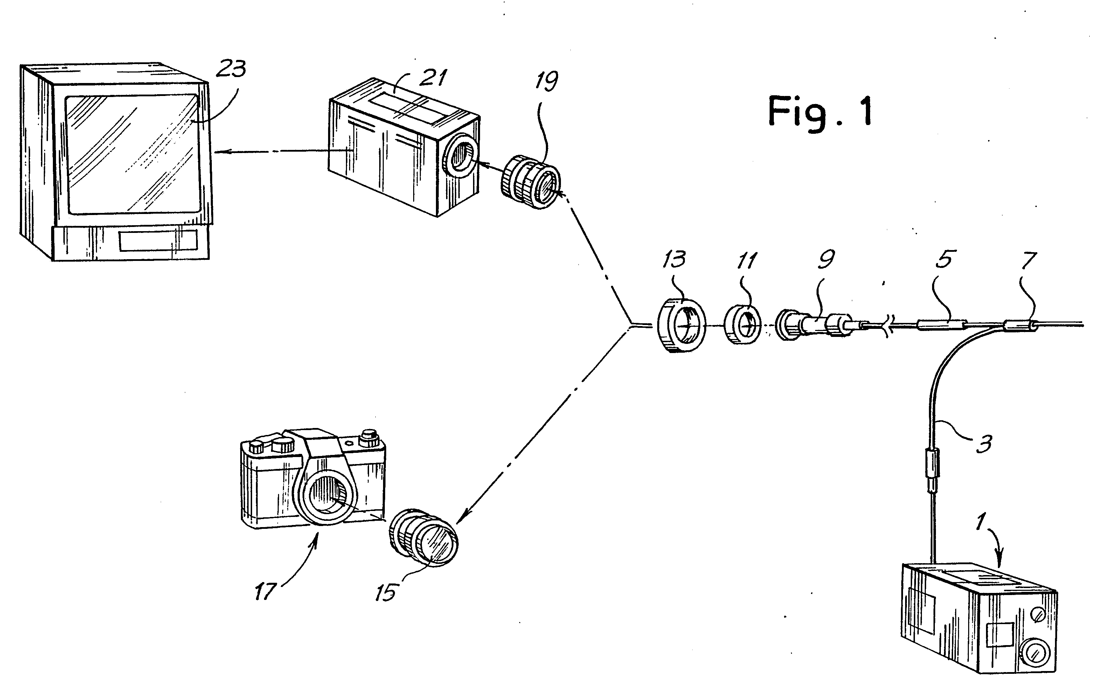

In Fig. 1, a diagram is shown of the complete equipment used by the endoscope according to the invention. According to the diagrammatic illustration in this figure, the equipment consists of a light source 1, for example a halogen lamp or similar, connected to the endoscope by means of a first bundle of optical fibers 3. Said optical fibers 3 constitute, together with a second bundle 5 for return of the image, a light guide 7, on the outside of which (not illustrated in this figure) a handle is applied, which is described in greater detail below.

The bundle of optical fibers 5 is connected to an ocular 9 which, by means of a system 11, 13 of flanges with a bayonet joint or similar, is connected to a lens 15 of a camera 17 or, alternatively, to a lens 19 of a telecamera 21 connected to a monitor 23. The camera 17 permits images to be photographed, for example for scientific use, whereas the telecamera and the monitor can be used during the work itself in order to check step by step the course of the work and/or the situation before and after the work itself.

All the equipment described above is of conventional type and already in use in the medical field and is not, therefore, described in greater detail.

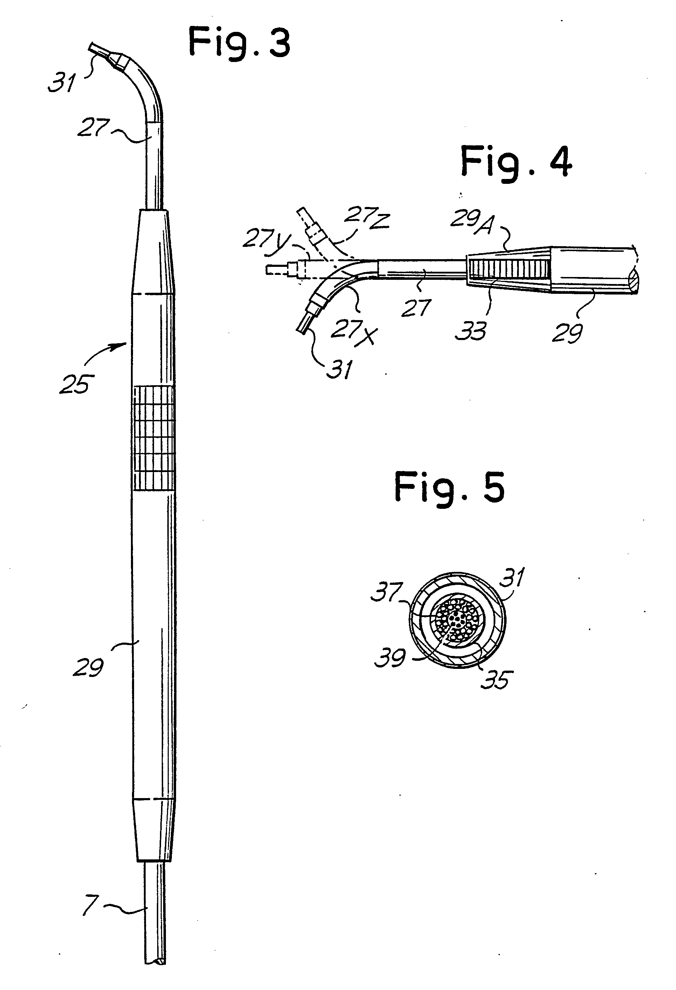

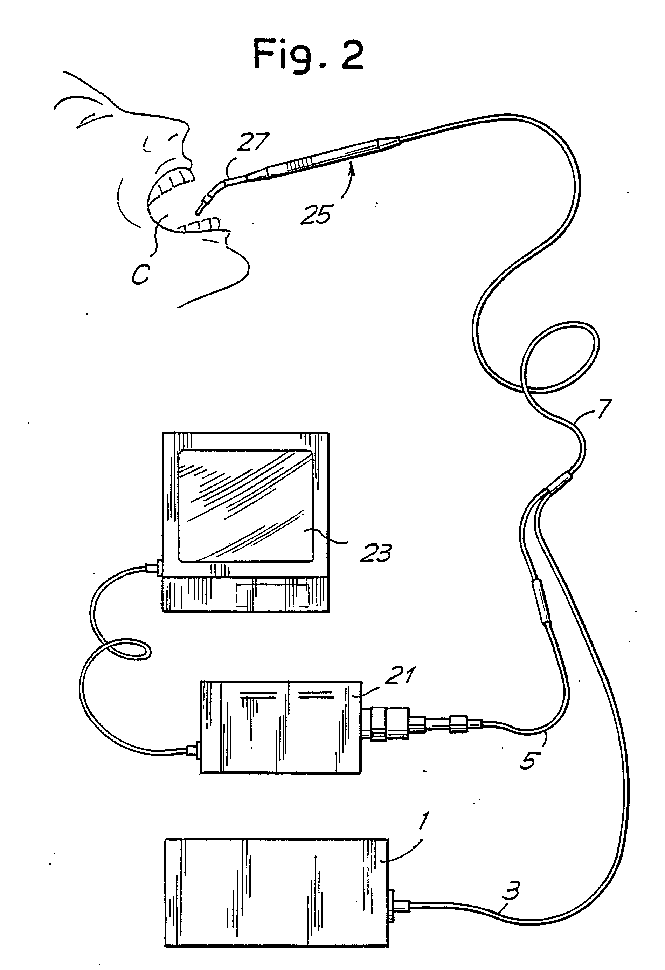

In Fig. 2, the same equipment as in Fig. 1 is shown diagramatically with only the telecamera 21 and the monitor 23. A handle 25 with an end part 27, which constitute the endoscope proper for the inspection of the oral cavity C of the patient, can also be seen in this figure.

The handle is illustrated in greater detail in Figs 3, 4 and 5. More specifically, Fig. 3 shows a lateral view of the handle 25 which consists of a body 29, which forms the grip of the handle, and which supports the end part 27; the latter is advantageously produced in the form of a small metal tube, which can be articulated in all directions, or in the form of a flexible element, in order to allow the doctor to vary its angular position as necessary. The end of the end part 27 consists on the other hand of a rigid tubular element 31, from which the two coaxial bundles of optical fibers look out towards the work area. The tubular element 31 has a very small diameter in order to allow convenient inspection even of the root canals or other areas of the oral cavity to which access is otherwise difficult. In particular, the diameter of said tubular element can be comprised, for example, between 1.5 and 0.5 mm and is preferably equal to 0.6 mm. In Fig. 4, three different possible positions, indicated respectively by 27X, 27Y, 27Z, of the end part 27 are shown. The possibility of varying the angular position of the end part 27 of the handle makes it possible to rotate the image visible on the monitor 23, keeping the handle 25 in a fixed position, or to proceed, for example, from an examination of the upper dental arch to the lower without rotating the axis of the bundle (and thus the image on the monitor), but by bending the end part 27, or vice versa. This is particularly advantageous as it allows the doctor to see the image on the monitor in exactly the same position as that in which he was accustomed to seeing it with the small mirror; this facilitates psychological adaptation to use of the new equipment as well as affording the device greater flexibility. Advantageously, on the conical part 29A of the handle, adjacent to the end part 27, four zones of different color can be arranged, one of which is indicated by 33 in Fig. 4. This expedient makes it possible to find and remember different angular positions of the end part 27, each colored zone corresponding to a different rotation of the image on the monitor in relation to the actual image.

Fig. 5 shows a cross-section of the tubular element 31 of the endoscope, from which the position of the two coaxial bundles of optical fibers can be seen. Inside the tubular element 31, a protective sheath 35 is arranged, which contains a first bundle of outer optical fibers 37 and a second bundle of inner optical fibers 39. The outer bundle 37 conveys the light for illumination of the area to be inspected, whereas the inner bundle 39 conveys the image collected in the observation zone to the ocular and the optics of the telecamera.

The end part of the endoscope may be interchangeable, for example, in order to use tubular elements 31 of different diameters to gain access in particular parts of the oval cavity.

## Claims

1. An endoscope for dental use, consisting of a handle (25) with an end part (27), to be introduced into the oral cavity, at the end (31) of which look out fiber-optical means for illumination of the area to be inspected and for collecting the image to be conveyed to means (17; 21, 23) for picking up the image.

2. The endoscope as claimed in Claim 1, wherein said end part (27) is curved.

3. The endoscope as claimed in Claim 1, wherein said end part (27) is flexible or articulated so as to assume different angular positions in relation to the axis of said handle (25).

4. The endoscope as claimed in Claim 3, wherein means (33) for determining the angular position assumed by said end part (27) in relation to said handle are provided on said handle.

5. The endoscope as claimed in one or more of the preceding Claims , wherein said fiber-optical means consist of a light guide with two bundles (37, 39) of optical fibers, a first bundle (37) conveying light from a light source to the area to be inspected and a second bundle (39) conveying the image from the area to be inspected to the means for picking up the image.

6. The endoscope as claimed in Claim 5, wherein said two bundles (37, 39) are coaxial, the second being inside the first.

7. The endoscope as claimed in one or more of the preceding claims, wherein the end part of the endoscope is interchangeable.

Fig. 1

EP 0 389 453 A1

# Fig. 3

*31*

*27*

*25*

*29*

*7*

# Fig. 4

*27z*

*27y*

*29A*

*27x*

*27*

*31*

*33*

*29*

# Fig. 5

*37*

*31*

*39*

*35*

# Fig. 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | DE-A-2208902 (RITTER A.G.)<br>* page 4, lines 2 - 25; claims 1, 2; figures *<br>--- | 1, 2, 5, 7 | A61B1/24<br>A61B1/04 |
| X | EP-A-280397 (E.L. ·ADAIR)<br>* column 4, line 14 - column 6, line 35 *<br>* column 8, line 15 - column 9, line 19; figures *<br>--- | 1-3, 5-7 | |
| X | US-A-2641977 (T. UJI ET AL.)<br>* column 2, lines 23 - 54; figures *<br>----- | 1-4 | |

|  | TECHNICAL FIELDS SEARCHED (Int. Cl.5 ) |
|---|---|
|  | A61B |

The present search report has been drawn up for all claims

| Place of search | Date of completion of the search | Examiner |
|---|---|---|
| THE HAGUE | 19 JUNE 1990 | RIEB K.D. |